# EUROPEAN PATENT APPLICATION

(11) **EP 1 902 734 A1**
(43) Date of publication of application: **26.03.2008**
(21) Application number: 06121180.1
(22) Date of filing: 25.09.2006
(51) Int. Cl.: A61K 47/48, A61K 38/04

(54) **uPAR selective contrast agent for magnetic resonance imaging**

(71) Applicant: Deutsches Krebsforschungszentrum Stiftung des öffentlichen Rechts, 69120 Heidelberg (DE)
(72) Inventor: Jugold, Manfred, 69214 Eppelheim (DE); Kießling, Fabian, 69121 Heidelberg (DE); Zhang, Chunfu, Heidelberg, 69120 (DE); Wängler, Björn, 69259 Wilhelmsfeld (DE); Pipkorn, Rüdiger, 69124 Heidelberg (DE); Semmler, Wohlfhard, 69118 Heidelberg (DE)
(74) Representative: Dick, Alexander

(57) **Abstract**

The present invention relates to a contrast agent for magnetic resonance imaging comprising a compound of the formula Pep-L-Im, wherein

Pep is a polypeptide molecule selected from the group consisting of
(i) polypeptide having an amino acid sequence of SEQ ID NO:1; SEQ ID NO:2; or SEQ ID NO:3;
(ii) polypeptide having an amino acid sequence which is at least 80 % identical with one of the amino acid sequences of SEQ ID NO:1; SEQ ID NO:2; or SEQ ID NO:3; and
(iii) polypeptide derivative obtained through substitution, addition, inversion and/or deletion of one or two amino acids of the amino acid sequences of SEQ ID NO:1; SEQ ID NO:2; or SEQ ID NO:3, which binds to uPAR;

L is a covalent single bond or a spacer; and
Im is at least one superparamagnetic iron oxide particle having a coating of an anorganic or organic or anorganic and organic polymer, wherein the coating is covalently attached to L or to Pep, when L is a bond.

Another aspect of the present invention is an in-vivo diagnostic method based on magnetic resonance imaging using said contrast agent.

SEQUENCE LISTING

<110>  Deutsches Krebsforschungszentrum (DKFZ)

<120>  uPAR selective contrast agent for magnetic resonance imaging

<130>  DK64425EP

<160>  6

<170>  PatentIn version 3.3

<210>  1
<211>  15
<212>  PRT
<213>  artificial sequence

<220>
<223>  uPAR binding peptides

<400>  1

Ser Glu Lys Thr Asn Arg Thr Leu Ser Tyr Arg Thr Gly Leu Lys
1               5                   10                  15


<210>  2
<211>  15
<212>  PRT
<213>  artificial sequence

<220>
<223>  uPAR binding peptides

<400>  2

Gln Gly Asn Ser Gly Arg Ala Val Thr Tyr Ser Arg Ser Arg Tyr
1               5                   10                  15


<210>  3
<211>  10
<212>  PRT
<213>  artificial sequence

<220>
<223>  uPAR binding peptides

<400>  3

Cys Asn Lys Tyr Phe Ser Asn Ile Cys Trp
1               5                   10


<210>  4
<211>  16
<212>  PRT
<213>  artificial sequence

<220>
<223>  uPAR binding peptides

<400>  4

Ser Glu Lys Thr Asn Arg Thr Leu Ser Tyr Arg Thr Gly Leu Lys Cys
1               5                   10                  15


<210>  5

<211>  16
<212>  PRT
<213>  artificial sequence

<220>
<223>  uPAR binding peptides

<400>  5

Gln Gly Asn Ser Gly Arg Ala Val Thr Tyr Ser Arg Ser Arg Tyr Cys
1               5                   10                  15


<210>  6
<211>  13
<212>  PRT
<213>  artificial sequence

<220>
<223>  uPAR binding peptides

<400>  6

Gly Gly Gly Cys Asn Lys Tyr Phe Ser Asn Ile Cys Trp
1               5                   10

## Description

The present invention relates to a contrast agent for magnetic resonance imaging as well as to an in-vivo diagnostic method based on magnetic resonance imaging using said contrast agent.

Powerful diagnostic imaging techniques using various contrast agents have been developed in recent years. One type of contrast agent includes a chelating molecule which is capable to coordinate an active metal for the desired imaging application. Such an application may be single-photon-emission computer tomography (SPECT) where a suitable metal is ¹¹¹In^{III}. Another method is called positron emission tomography (PET) where normally ⁶⁸Ga^{III} is used as an active metal. Finally, magnetic resonance imaging (MRI) has to be named as a useful method where Gd^{III} is coordinately bound to a chelator in a contrast agent. MRI offers a high spacial resolution and good contrast results for soft tissue. Another important radio imaging technique is scintigraphy.

To improve the efficacy of contrast agents, attempts were made to selectively address distinct cells, tissue or organs which show pathological mutations.

An interesting target in this regard is the urokinase-type plasminogen activator receptor (uPAR). It is believed that this receptor can be used as an indicator for the monitoring of diseases or disorders associated with inflammation, like arthritis, arteriosclerosis, and cancer.

A. P. Masar, Anti-cancer drugs 12 (2001), 387-400, describes for example the use of urokinase plasminogen activator receptor (uPAR) as a target for the diagnosis and therapy of cancer.

The role of uPAR in inflammatory processes is described by D. Gveric et al., Brain 124 (2001), 1978-1988.

The importance of the uPA/uPAR systems for the development of arteriosclerosis and restinosis is described by A. Kusch et al., Z. Kardiol 90 (2001), 307-318.

The same system as a constitutive component of the inflamed synovial fluid which induces arthritis is described by T. Jin et al., Arthritis Res. Ther. 5 (2003), R9-R17.

The use of uPAR-targeting cyclic peptides which selectively bind to uPAR and derivatives thereof for a use in positron emission tomography (PET) is described in WO-A 00/26353.

However, contrast agents which selectively bind to uPAR for magnetic resonance imaging are not described so far.

Thus, an object of the present invention is to provide a uPAR selective contrast agent which can be used in magnetic resonance imaging.

Accordingly, the present invention provides a contrast agent for magnetic resonance imaging comprising a compound of the formula Pep-L-Im, wherein

Pep is a polypeptide molecule selected from the group consisting of
(i) polypeptide having an amino acid sequence of SEQ ID NO:1; SEQ ID NO:2; or SEQ ID NO:3;
(ii) polypeptide having an amino acid sequence which is at least 80 % identical with one of the amino acid sequences of SEQ ID NO:1; SEQ ID NO:2; or SEQ ID NO:3; and
(iii) polypeptide derivative obtained through substitution, addition, inversion and/or deletion of one or two amino acids of the amino acid sequences of SEQ ID NO:1; SEQ ID NO:2; or SEQ ID NO:3, which binds to uPAR;
   L is a covalent single bond or a spacer; and
   Im is at least one superparamagnetic iron oxide particle having a coating of an anorganic or organic or anorganic and organic polymer, wherein the coating is covalently attached to L or to Pep, when L is a bond.

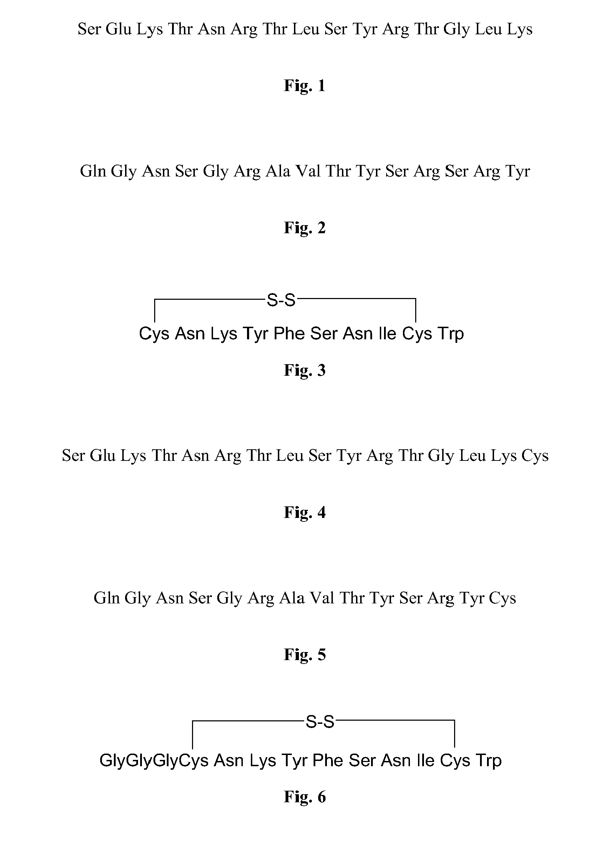

It was surprisingly found that the amino acids of SEQ ID No. 1, SEQ ID No. 2 and SEQ ID No. 3 as shown in Figures 1-3 are especially suitable in combination with coated supermagnetic iron oxide particles for magnetic resonance imaging.

Magnetic resonance imaging (MRI) is well known in medical diagnostics. In a strong magnetic field radio-frequency (rf) pulses are used to excite free protons in tissue. After rf excitation relaxation of the magnetization occurs in two different ways. Depending on tissue properties those two effects are described by the time constants, longitudinal (T₁) and transversal (T₂) relaxation time. Usually liquid parts of the tissue are hyperintense in T₂-weighted MR images and hypointense in T₁-weighted MR images. Fatty tissue is hyperintense in both methods. Due to local edema pathologies are often better assessed by T₂-weighted techniques. Thus, the morphological assessment is often better on the T₁-weighted images.

The contrast agent for magnetic resonance imaging according to the present invention comprises a compound of the formula Pep-L-Im.

Pep is a polypeptide molecule which is capable to bind to uPAR. Thus, it is possible to selectively address the uPA receptor with a contrast agent of the present invention.

Suitable polypeptides are those which have an amino acid sequence of SEQ ID No. 1, SEQ ID No. 2 or SEQ ID No. 3.

The polypeptide of SEQ ID No. 3 is a cyclic peptide, wherein the thio groups of the two cysteine units are connected to yield a disulfide bridge.

Additionally, polypeptides can be used which are at least 80 % identical with one of the above mentioned amino acid sequences.

In the context of the present invention the expression "at least 80 % identical" preferably refers to agreement at the level of the amino-acid sequence which may be determined by means of recognized procedures, e.g. computer-generated sequence comparisons (Altschul, S. F., Gish, W., Miller, W., Myers, E. W. and Lipman, D. J., J. Mol. Biol. 215 (1990), 403-410). The expression "identity" or "identical" here signifies the degree of relationship between two or more amino acid molecules as determined through the agreement between the sequences, in which under agreement both identical agreement and conservative amino-acid replacement is to be understood. The percentage of "identity" is indicated by the percentage of identical regions in two or more sequences taking into consideration gaps and other sequence particulars.

The concept "conservative amino-acid replacement" refers to a replacement of one amino-acid residue by another amino-acid residue, in which the replacement should exert the most limited possible influence on the (spatial) structure of the polypeptide molecule. Fundamentally four physico-chemical groups are distinguished, into which the naturally occurring amino-acids are divided. Arginine, lysine and histidine belong to the basic amino-acid group. To the acidic amino-acids belong glutamic acid and aspartic acid. The chargeless/polar amino-acids consist of glutamine, asparagine, serine, threonine and tyrosine. The non-polar amino-acids comprise phenylalanine, tryptophane, cysteine, glycine, alanine, valine, methionine, isoleucine, leucine and proline. In this context a conservative amino-acid replacement means the replacement of an indicated amino-acid by an amino-acid belonging to the same physico-chemical group.

The identity of polypeptide molecules related to one another can be determined with the aid of recognized procedures. Preferred procedures for the determination of identity lead most closely to the greatest agreement between the sequences investigated. Computer programmes for the determination of identity between two sequences include the GCG programme package, comprehending GAP (Devereux, J., et al., Nucleic Acids Research 12 (12) 387, 1984), Genetics Computer Group University of Wisconsin, Madison Wis.; BLASTP, BLASTN and FASTA but are not restricted to these. The BLASTX programme can be obtained from the National Centre for Biotechnology Information (NCBI) and from other sources (BLAST handbook, Altschul S. et al, NCB NLM NIH Bethesda Md. 20894). The well-known Smith Waterman algorithm can also be used for the determination of identity.

The GAP programme is also suitable for use with the foregoing parameters. Further exemplary algorithms, gap opening penalties, gap extension penalties, and comparison templates including that named in the programme handbook, Wisconsin package, version 9, September 1997, may be used. The choice is dependent on the comparison being carried out and in addition on whether the comparison is being done between two sequence pairs, for which GAP or Best Fit are preferred, or between a sequence and a comprehensive data-bank, for which FASTA or BLAST are preferred.

An agreement of 80 % obtained with the above-named algorithm is, within the meaning of the present invention, preferably taken as 80 % identity. Higher grades of identity are correspondingly valid.

More preferred the polypeptide of (ii) has an amino acid sequence which is at least 85 % identical, even more preferred at least 90 % identical, even more preferred 95 % identical.

Useful polypeptide molecules are also derivatives which can be obtained through substitution, addition, inversion and/or deletion of 1 or 2 amino acids of the above mentioned amino acid sequences of SEQ ID No. 1; SEQ ID No. 2; or SEQ ID No. 3, which bind to uPAR.

The term "polypeptide" within the meaning of the present invention relates to a peptide having a sequence of at least 6 amino acids. Preferably, the amino acids are selected from the group of amino acids selected from natural occurring amino acids.

Since the polypeptides which are suitable as group "Pep" have a relatively short number of amino acids constituting the amino acid sequence, they can be synthesized using conventional solid phase peptide synthesis (SPPS). Such protocols for the preparation of polypeptides are well known in the art and can be used for the purposes of the present invention.

Binding assays for the determination of the specific or non-specific binding to uPAR are also well known in the art and can be used to identified polypeptide derivatives of sequences according to SEQ ID No.: 1, SEQ ID No.: 2 or SEQ ID No.: 3, which bind selectively to uPAR.

A multitude of amino acid compounds can be synthesized by combinatorial chemistry and tested using parallel high throughput screening systems like phage display or microarray screening.

An exemplary label-free detection method for the parallel detecting of binding events is surface plasmon resonance. A suitable apparatus for a parallel testing is disclosed in WO-A 01/63256.

The polypeptide molecules are directly attached to the imaging moiety of the compound comprised in the contrast agent according to the present invention or are spaced apart by a spacer. Thus, the group L is a covalent single bond (in case of direct attachment) or a spacer.

In general, any organic molecule is capable to serve as a spacer as long as resulting molecule is capable of binding to the uPAR and is suitable for magnetic resonance imaging.

Preferably, L is a spacer comprising at least one of the following:
(a) a polypeptide of 1 to 10 amino acids;
(b) polyethylene glycol;
(c) an C₁₋₁₀ alkyl chain which is optionally interrupted by one or more atoms or groups independently selected from the group consisting of oxygen; sulphur; nitrogen (NH or N(C₁₋₄ alkyl)); heterocycle; C₃₋₇ cycloalkyl; and phenyl adn optionally substituted with one or more substituents independently selected from the group consisting of OH; =O; OC₁₋₆ alkyl; C(O)OH; C(O)OC₁₋₆ alkyl; OC(O)C₁₋₆ alkyl; SH; =S; SC₁₋₆ alkyl; C(O)SH; C(O)SC₁₋₆ alkyl; SC(O)C₁₋₆ alkyl; NH₂; NH(C₁₋₆ alkyl); N(C₁₋₆ alkyl)₂; C(O)NH₂; C(O)NH(C₁₋₆ alkyl); C(O)N(C₁₋₆ alkyl)₂; NHC(O)C₁₋₆ alkyl; N(C₁₋₆ alkyl)C(O)C₁₋₆ alkyl; and halogen.

Preferably, the polypeptide which can serve as group L or is included in L has 1, 2 or 3 amino acids. More preferred, the amino acids are selected from the group consisting of cysteine and glycine. In a most preferred embodiment Pep-L is selected from the group of amino acids selected from the group of sequences SEQ ID No.: 4 (Fig. 4), 5 (Fig. 5) and 6 (Fig. 6).

The peptide of sequence SEQ ID No. 6 is a cyclic peptide, wherein the thio groups of the two cysteine units are connected to yield a disulfide bridge.

It is preferred that L consists of at least one of the aforementioned alternatives (a), (b) or (c).

Within the meaning of the present invention the terms are used as follows:

"Alkyl" means a straight-chain or branched carbon chain that may contain double or triple bonds. It is generally preferred that alkyl doesn't contain double or triple bonds. Each hydrogen of an alkyl carbon may be replaced by a substituent.

"C₁₋₄ alkyl" means an alkyl chain having 1 - 4 carbon atoms, e.g. if present at the end of a molecule: methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl, or e.g. -CH₂-, -CH₂-CH₂-, -CH=CH-, -CH(CH₃)-, -C(CH₂)-, -CH₂-CH₂-CH₂-, -CH(C₂H₅)-, -CH(CH₃)₂-, when two moieties of a molecule are linked by the alkyl group. Each hydrogen of a C₁₋₄ alkyl carbon may be replaced by a substituent.

"C₁₋₆ alkyl" means an alkyl chain having 1 - 6 carbon atoms, e.g. if present at the end of a molecule: C₁₋₄ alkyl, methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl; tert-butyl, n-pentane, n-hexane, or e.g. -CH₂-, -CH₂-CH₂-, -CH=CH-, -CH(CH₃)-, -C(CH₂)-, - CH₂-CH₂-CH₂-, -CH(C₂H₅)-, -CH(CH₃)₂-, when two moieties of a molecule are linked by the alkyl group. Each hydrogen of a C₁₋₆ alkyl carbon may be replaced by a substituent.

"C₁₋₁₀ alkyl" means an alkyl chain having 1-10 carbon atoms, e.g. C₁₋₄ alkyl, C₁₋₆ alkyl, heptane, octane, nonane, decane.

"C₃₋₇ cycloalkyl" or "C₃₋₇ cycloalkyl ring" means a cyclic alkyl chain having 3 - 7 carbon atoms, e.g. cyclopropyl, cyclobutyl, cyclopentyl, cyclohexyl, cyclohexenyl, cycloheptyl. Each hydrogen of a cycloalkyl carbon may be replaced by a substituent.

"Halogen" means fluoro, chloro, bromo or iodo. It is generally preferred that halogen is fluoro or chloro.

"Heterocyclyl" or "heterocycle" means a cyclopentane, cyclohexane or cycloheptane ring that may contain up to the maximum number of double bonds (aromatic or non-aromatic ring which is fully, partially or un-saturated) wherein at least one carbon atom up to 4 carbon atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a heterocycle are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine or homopiperazine. "Heterocycle" means also azetidine.

As far as the spacer L is a polypeptide, it is preferred that the sequence of the polypeptide consists of the group of natural occurring amino acids, i.e. alanine, arginine, asparagine, aspartic acid, cysteine, glutamine, glutamic acid, glycine, histidine, isoleucine, leucine, lysine, methionine, phenylalanine, proline, serine, threonine, tryptophane, tyrosine and valine.

The moiety Im is at least one superparamagnetic iron oxide particle having a coating of an anorganic or organic or anorganic and organic polymer wherein the coating is covalently attached to L or to Pep, when L is a bond.

In this context, surface coating of USPIO (ultra small superparamagnetic iron oxid) nanoparticles using silica and alkoxysilanes is promising (Coradin, T.; Lopez, P. J. Chembiochem. 2003, 4, 251-259). Silica and alkoxysilanes are among the most frequently used surface coating materials for inorganic nanoparticles, especially iron oxide particles (Philipse, A. P.; van Bruggen, M. P.; Pathmamanoharan, C. Langmuir 1994, 10, 92-99. Yang, H. H.; Zhang, S. Q.; Chen, X. L.; Zhuang, Z. X.; Xu, J. G.; Wang, X. R. Anal. Chem. 2004, 76, 1316-1321. Bruce, I. J.; Sen, T. Langmuir 2005, 21, 7029-7035. Kohler, N.; Fryxell, G. E.; Zhang, M. J. Am. Chem. Soc. 2004, 126, 7206-7211. Mikhaylova, M.; Kim, D. K.; Berry, C. C.; Zagorodni, A.; Toprak, M.; Curits, A. S.; Muhammed, M. Chem. Mater. 2004, 16, 2344-2345. Fan, H.; Chen, Z.; Brinker, C. J.; Clawson, J.; Alam, T. J. Am. Chem. Soc. 2005, 127, 13746-13747. Donselaar, L. N.; Philipse, A. P.; Suurmond, J. Langmuir 1997, 13, 6018. Liu, Q.; Xu, Z.; Finch, J. A.; Egerton, R. Chem. Mater. 1998, 10, 3936. Correa-Duarte, M. A.; Giersig, M.; Kotov, N. A.; Liz-Marzan, L. M. Langmuir 1998, 14, 6430). Furthermore, the surface of silica coated particles is often terminated by a silanol group that can react with various coupling agents to covalently attach specific ligands (Ulman, A. Chem. ReV. 1996, 96, 1533). Alkoxysilanes are bifunctional molecules with the general formula X-(CH₂)ₙSiR (OR')₃₋ₙ, where X represents the headgroup functionality, (Ch₂)ₙ a flexible spacer, and Si(OR)ₙ the anchor groups by which they can attach to free Si-OH or Fe-OH surface groups after hydrolysis of the alkoxy group (Bruce, I. J.; Sen, T. Langmuir 2005, 21, 7029-7035). In contrast to silica coatings, which need further surface modification for coupling bioactive molecules, this can be done directly using alkoxysilanes with functional groups, such as amino groups.

Silica and alkoxysilanes coated iron oxide particles were used as carriers of enzymes to investigate biologic catalysis (Yang, H. H.; Zhang, S. Q.; Chen, X. L.; Zhuang, Z. X.; Xu, J. G.; Wang, X. R. Anal. Chem. 2004, 76, 1316-1321. Yang, X.; Li, Z.; Liu, B.; Klein-Hofmann, A.; Tian, G.; Feng, Y.; Ding, .Y; Su, D.; Xiao, F. Adv. Mater. 2006, 18, 410-414), to perform magnetic bioseparation (Bruce, I. J.; Sen, T. Langmuir 2005, 21, 7029-7035) and to label epithelial cells in vivo (Dormer, K.; Seeney, C.; Lewelling, K.; Lian, G.; Gibson, D.; Johnson, M. Biomaterials 2005, 26, 2061-2072). There was also report on the principle possibility to use silica-imbedded iron oxide nanoparticles as contrast agent in MRI (Yan, F.; Xu, H.; Anker, J.; Kopelman, R.; Ross, B.; Rehemtulla, A.; Reddy, R. J Nanosci. Nanotechnol. 2004, 4, 72-76).

Furthermore, suitable coatings are those of carboxy polysaccharides. Those coated particles are disclosed in EP-B 656368 or EP-A 516252.

Thus, in a preferred embodiment of the present invention the coating comprises silica, a carboxy polysaccharide or an alkoxysilane. Furthermore, it is preferred that the coating consists of one of these alternatives.

Furthermore, it is preferred that the silanes are selected from the group consisting of 3-aminopropyltrimethoxysilane (APTMS); and N-(2-aminoethyl)-3-aminopropyltrimethoxysilane (AEAPTMS).

It is preferred that L is attached to the C- or N-terminal end of Pep.

Suitable iron oxide particles sizes are known in the art. Preferably, the particle size with coating is less than 60 nm. Furthermore, it is preferred that the isoelectric point of the particle is between 5.0 and 10, more preferred about 7.4. Preferably, the thickness of the coating is small compared to the particle size of the iron particle without coating. In a preferred embodiment the coating has a thickness of less than 10 nm.

Preferably, the iron oxide particles have a size of less than 10 nm.

Preferably the iron particles are additionally coated with polyethylene glycol (PEG) to prevent or decrease the uptake of the particles reticuloendothelial system.

For the preparation of USPIO, a modified Massart method can be used.

In a first step a mixture of FeCl₃ and FeCl₂ in hydrochloric acid can be added to a solution of sodium hydroxide under an inert nitrogen atmosphere.
For coating, USPIO particles are dispersed into an appropriate solvent, like toluene and the coating material is added the suspension. The ferrofluid suspension is then refluxed under mechanical stirring. The modified particles can be collected magnetically and redispersed into methanol by ultrasonication.

Another aspect of the present invention is an in-vivo diagnostic method based on magnetic resonance imaging for detecting the presence of uPAR on the surface of a cell, in a tissue, in an organ or in a biological sample, which cell, tissue, organ or sample is suspected of expressing uPAR due to a pathological state, comprising
(a) contacting said cell, tissue, organ or sample with a contrast agent of the present invention; and
(b) detecting the superparamagnetic iron oxide particle associated with said cell, tissue, organ or sample by magnetic resonance imaging.

Preferably, the pathological state is associated with inflammation, like arthritis, arteriosclerosis, cancer or combinations thereof.

### Examples

### Example 1

### Synthesis of Superparamagnetic Iron Oxide Particles (USPIO)

### Materials and Methods

Ferric chloride (FeCl₃, > 97 %), ferrous chloride tetrahydrate (FeCl₂ · 4H₂O, > 99%), sodium hydroxide solution (1 M), sodium metasilicate pentahydrate (Na₂O₃Si · 5H₂O), hydrochloric acid (HCl, 1 M solution), methanol (> 99,8 %), toluene (> 99,8 %), 3-aminopropyltrimethoxysilane (APTMS, > 97 %), N-(2-aminoethyl)-3-aminopropyltrimethoxysilane (AEAPTMS, > 98 %), nucleus fast red and Prussian blue were purchased from Sigma-Aldrich (St. Louis, MO). Milli-Q (U.S.A) purified water was used for all experiments.

### Synthesis and Characterization

### Synthesis of Ultra Small Superparamagnetic Iron Oxide Nanoparticles (USPIO):

For the preparation of USPIO, a modified Massart method was used (Yan, F. et al., J. Nanosci. Nanotechnol. 2004, 4, 72-76). A mixture of FeCl₃ (20 mL, 1 M in 0.2 M HCl) and FeCl₂ (20 mL, 0.5 M in 0.2 M HCl) was added dropwise to a mechanically stirred solution of sodium hydroxide (200 mL, 1.5 M) within 5 minutes under an inert nitrogen atmosphere at 80 °C. The mixture was stirred for 20 minutes and subsequently cooled down to ambient temperature. The sediment was separated by an external magnetic field, washed three times with deionized water and dispersed into 100 mL aqueous hydrochloride acid (0.01 M).

### Surface Modification of USPIO with silica, 3-aminopropyltrimethoxysilane (APTMS) and N-(2-aminoethyl)-3-aminopropyltrimethoxysilane (AEAPTMS):

Using sodium metasilicate pentahydrate as silica source, a thin layer of silica could be deposited onto the USPIO (A. P. Philipse et al., Langmuir 1994, 10, 92-99). Briefly, a solution of 200 mL 0.58 wt% SiO₂ (from Na₂O₃Si · 5H₂O) was passed through a Dowex 50 Wx4 acid ion exchange column which was previously regenerated by subsequent flushing with hot double-distilled water (about 100 °C), 3 M HCl and cooled to ambient temperature using double-distilled water. The USPIO (1 g) were dispersed into the eluent after adjusting the pH value to 9.5 with 0.5 M sodium hydroxide solution. The suspension was incubated at 80 °C for 2 hours with vigorous mechanical stirring. The coated particles were separated with the aid of a magnet and washed three times with deionized water. Finally, the particles were dispersed into water.

Thin silane coating was achieved by a direct silanizing of the USPIO with APTMS (M. Mikhaylova et al., Chem. Mater. 2004, 16, 2344-2345). USPIO (1 g) were first washed with methanol (50 mL), thereafter with a mixture of methanol and toluene (50 mL, v/v = 1:1) and finally with toluene alone (50 mL). Subsequently, the particles were dispersed into toluene (100 mL) and APTMS (0.1 mL, 5.73 mol/L) was added dropwise to the suspension within 5 minutes. The ferrofluid suspension was transferred into a three-necked flask with N₂ flow and refluxed under mechanical stirring for 10 h. The modified particles were magnetically collected and redispersed into methanol by ultrasonication. After washing with methanol the APTMS-coated USPIO particles were dispersed into water.

The process for AEAPTMS coating was analogous, with the exception that AEAPTMS was added to the USPIO-toluene-suspension to achieve a final concentration of 4.6 mM.

### Example 2

### Additional PEG-coating

In order to reduce the unspecific phagocytosis and prolong the half life of the probe in vivo, further modification of the particles with functional polyethylene glycol (PEG) could be performed. The functional PEG has a Boc- or Fmoc-protected amino group at one end and carboxylic group or N-hydrosuccimide (NHS) group at the other end. The molecular weight of PEG could be varied between 100 and 5000.

For modification of the particles with carboxylic group ended PEG, the following procedure may be used:

1-Ethyl-3-(dimethylaminopropyl)carbodiimide hydrochloride (EDC, 0.4 mg) and N-hydrosuccinimide (NHS, 0.6 mg) were added to 1 mL 2-(N-morpholino)ethane sulfonic acid (MES) buffer (0.1 M, pH 4.5 ∼ 6). Two-hundred microgram of PEG were added to 300 µl of the mixture and incubated for fifteen minutes at room temperature. Twenty milligram of (AEAPS- or AEAPTMS-coated) USPIO were dispersed into the solution and incubated for 60 min at room temperature. Then, the USPIO were separated with a magnet and washed three times with PBS.

For modification of the particles with NHS-ended PEG, the following procedure may be used: Ten milligram of NHS-ended PEG is dissolved into 0.5 ml deioned water. Subsequently, ten milligram of (AEAPS- or AEAPTMS-coated) USPIO were dispersed. The suspension is vortexed and stirred for two hours at room temperature. PEG-coated USPIO were washed three times with deioned water.

The BOC-protected amino group can be deprotected by incubating the particles in acid medium. For deprotection of Fmoc-protected amino group, incubate the particles with 20 % of pepridine in DMF for 20 min and 50 % pepridine in DMF for 30 min successively. After deprotection, the free amino group on the surface of particles can be used for bioconjugation.

### Example 3

### Coupling of uPAR specific peptides to USPIO

1-10 milligram EDC and NHS were weighed into 300 µl 0.1 M PBS (phosphate-buffered saline, pH 7.4). Two hundred microgram of the peptides according to amino acid sequences SEQ ID No.: 4, 5 and 6 (Fig. 4-6) were added and incubated for five minutes at 4 °C. Twenty milligram of USPIOs were dispersed into the solution and incubated for 30 min at room temperature.

### Example 4

For an in vitro study RKO cells are used which are characteristic for expressing and presenting uPAR on the cell surface. RKO is a poorly differentiated colon carcinoma cell line lacking h-TRbeta1 (Boyd D, et al. (1988). Cancer Res 48:3112-3116).
For measuring the in vitro uptake of unlabeled and peptide labeled USPIO the particles are added in a concentration of 0.03 µmol/ml iron to the growth medium of the RKO cells. Before adding the USPIOs, cells are preincubated for five minutes with plain medium or with 10.000 fold excess of uncoupled peptides. All competition experiments are carried out for 1 hour to reduce time dependent unspecific particle uptake.
After incubation the cells are resuspended in gelantine (2x10⁷ cells /ml). Cell viability is evaluated by Trypan-Blue exclusion assay. To determine the USPIO uptake by the cells T₂-relaxation times are measured using a clinical 1.5 Tesla MR-scanner (Siemens Symphony). (CPMG-sequence: TR 1200 ms, TE range 7.4 - 236.8 ms, 32 echoes, Slice thickness 5.1 mm, 4 averages, voxel size 1.9 x 1.4 x 10 mm, 3.11 min acquisition time). T₂-Relaxation rates are determined.
Both, the targeted and non targeted USPIO do not show significant cell toxicity.
As compared with cell-suspensions incubated with unlabeled USPIOs, the uptake of uPAR-specific USPIOs and nonsense peptide labeled USPIOs increase T₂-relaxation rates by 345 % and 190 %, respectively. Competition of the uptake of uPAR-specific USPIOs by adding free peptides (10.000 fold excess) is successful and reduces the increase of T₂-relaxation rate by 45 %. As expected, uptake of nonsense peptide labeled USPIOs can not be competitively inhibited.

## Claims

1. A contrast agent for magnetic resonance imaging comprising a compound of the formula Pep-L-Im, wherein
Pep is a polypeptide molecule selected from the group consisting of
(i) polypeptide having an amino acid sequence of SEQ ID NO:1; SEQ ID NO:2; or SEQ ID NO:3;
(ii) polypeptide having an amino acid sequence which is at least 80 % identical with one of the amino acid sequences of SEQ ID NO:1; SEQ ID NO:2; or SEQ ID NO:3; and
(iii) polypeptide derivative obtained through substitution, addition, inversion and/or deletion of one or two amino acids of the amino acid sequences of SEQ ID NO:1; SEQ ID NO:2; or SEQ ID NO:3, which binds to uPAR;
L is a covalent single bond or a spacer; and
Im is at least one superparamagnetic iron oxide particle having a coating of an anorganic or organic or anorganic and organic polymer, wherein the coating is covalently attached to L or to Pep, when L is a bond.

2. The contrast agent of claim 1, wherein the polypeptide of (ii) has an amino acid sequence which is at least 85 % identical.

3. The contrast agent of claim 1 or 2, wherein L is a spacer comprising at least one of the following:
(a) a polypeptide of 1 to 10 amino acids;
(b) polyethylene glycol;
(c) an C₁₋₁₀ alkyl chain which is optionally interrupted by one or more atoms or groups independently selected from the group consisting of oxygen; sulphur; nitrogen (NH or N(C₁₋₄ alkyl)); heterocycle; C₃₋₇ cycloalkyl; and phenyl adn optionally substituted with one or more substituents independently selected from the group consisting of OH; =O; OC₁₋₆ alkyl; C(O)OH; C(O)OC₁₋₆ alkyl; OC(O)C_{1- 6} alkyl; SH; =S; SC₁₋₆ alkyl; C(O)SH; C(O)SC₁₋₆ alkyl; SC(O)C₁₋₆ alkyl; NH₂; NH(C₁₋₆ alkyl); N(C₁₋₆ alkyl)₂; C(O)NH₂; C(O)NH(C₁₋₆ alkyl); C(O)N(C₁₋₆ alkyl)₂; NHC(O)C₁₋₆ alkyl; N(C₁₋₆ alkyl)C(O)C₁₋₆ alkyl; and halogen.

4. The contrast agent of claim 3, wherein the polypeptide has 1 to 3 amino acids.

5. The contrast agent of claim 3 or 4, wherein the amino acid is selected from the group consisting of the natural occurring amino acids.

6. The contrast agent of any of claims 1 to 5, wherein L is attached to the C- or N-terminal end of Pep.

7. The contrast agent of any of claims 1 to 6, wherein the coating comprises silica, a carboxypolysaccharide or an alkoxysilane.

8. The contrast agent of claim 7, wherein the coating comprises at least one of the silanes selected from the group consisting of 3-aminopropyltrimethoxysilane; and N-(2-aminoethyl)-3-aminopropyltrimethoxysilane.

9. The contrast agent of any of claims 1 to 8, wherein the iron oxide particle has a size of less than 60 nm.

10. An in-vivo diagnostic method based on magnetic resonance imaging for detecting the presence of uPAR on the surface of a cell, in a tissue, in an organ or in a biological sample, which cell, tissue, organ or sample is suspected of expressing uPAR due to a pathological state, comprising
(a) contacting said cell, tissue, organ or sample with a contrast agent of any of claims 1 to 9; and
(b) detecting the superparamagnetic iron oxide particle associated with said cell, tissue, organ or sample by magnetic resonance imaging.
